Europäisches Patentamt

European Patent Office

Office européen des brevets

(19)

(11) Publication number: **0 167 340 B1**

(12) **EUROPEAN PATENT SPECIFICATION**

(45) Date of publication of patent specification: **09.09.92**

(51) Int. Cl.⁵: **G01N 33/53**, G01N 33/68, G01N 33/76, G01N 33/573, G01N 33/574, G01N 33/561

(21) Application number: **85304521.9**

(22) Date of filing: **25.06.85**

The file contains technical information submitted after the application was filed and not included in this specification

(54) **Method for assaying a biocomponent.**

(30) Priority: **26.06.84 JP 131556/84**

(43) Date of publication of application:
**08.01.86 Bulletin 86/02**

(45) Publication of the grant of the patent:
**09.09.92 Bulletin 92/37**

(84) Designated Contracting States:
**CH DE FR GB IT LI SE**

(56) References cited:
**EP-A- 0 060 518**
**FR-A- 2 273 280**

**IGAKUNA AYUMI, vol.31, no.6, 1984; pp.384-388 (Chemical Abstracts, Vol. 102, abstract no. 75036z)**

**ELECTROPHORESIS '83, Walter de Gruyter & Co. Berlin-New York, 1984**

(73) Proprietor: **Kyowa Medex Co. Ltd.**
**6-1 Ohtemachi Itchome Chiyoda-ku Tokyo(JP)**

(72) Inventor: **Ishiguro, Tatsuya**
**57, Shimogamominakuchi-machi Sakyo-ku Kyoto-shi Kyoto-fu(JP)**

(74) Representative: **Lambert, Hugh Richmond et al**
**D. YOUNG & CO. 10 Staple Inn London, WC1V 7RD(GB)**

EP 0 167 340 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to an improved method of conducting an immunoelectrophoresis assay, and especially for use in the diagnosis of cancer.

The following general methods are known for the detection of a biocomponent, for example, serum protein in a sample: colour-staining, silver staining, autoradiography and immunological methods using a flurorimmunoassay (FIA), radioimmunoassay (RIA), all of which have been used from time to time in the diagnosis of cancer.

The colour-staining method gives high backgrounds, unclear results and poor detection sensitivity.

Silver staining and autoradiography are high in sensitivity and accuracy, but are operationally complicated and cannot be carried out promptly and simply. There are also waste disposal problems and their applicability is limited.

The immunological methods can specifically detect single substances and have high sensitivity, give good quantitative determination and are of wide applicability. However, radioimmunoassays have a complicated operational procedure and are not applicable to double staining techniques and are thus of limited application.

Enzyme immunoassays, in general, have high sensitivity and clarity and are comparatively simple and rapid to carry out. However, conventional methods for colouring enzyme-labelled gel immunoelectrophoresis assays, using an enzyme-labelled antibody, have problems of high background, lack of clarity and low sensitivity.

Also, when carrying out gel immuno-electrophoresis assays a control marker or standard is generally made to migrate in the gel in a parallel but separate track. This method causes problems in the measurement of migration distances with any accuracy.

In Electrophoresis 1983, 4, 371-373 Takeda et al disclose the results of a study carried out on the separation and identification of different molecular species of human α-fetoprotein (AFP) in body fluid samples by double affinity electrophoresis with concavalin A and Lens cullinaris haemagglutinin A (LcH-A). It is not specifically stated, but it is known that the method of Takeda et al employs 4-chloro-1-naphthol as the chromogen in the subsequent development of the gel. In contrast to the method of Takeda et al, the present invention employs 4-methoxy-1-naphthol as the chromogen, it having been found by the present inventors that 4-methoxy-1-naphthol provides a much shorter colouring and migration time, a clearer migration diagram, and a markedly high sensitivity and resolving power as compared with 4-chloro-1-naphthol.

4-methoxy-1-naphthol is a known reagent for assaying hydrogen peroxide (EP-A-0060518) but it is not suggested that that chromogen is of any particular benefit in any particular application when compared either with 4-chloro-1-naphthol or any other known chromogen for the assay of hydrogen peroxide.

Thus, in accordance with the present invention an assay method is provided in accordance with the preamble to claim 1, and wherein there is used in the development of gel after electrophoresis, a chromogenic composition comprising the combination of hydrogen peroxide and 4-methoxy-1-naphthol.

In a more particular and preferred aspect of the present invention, there is added to the sample prior to electrophoresis, as an internal standard, a coloured protein-dye conjugate, the protein component of which has a different antigenic specificity from that of the biocomponent or target substance to be assayed, and there is incorporated into the gel-like carrier a receptor substance capable of undergoing a specific binding reaction with the standard protein. By adding a control marker combined with a colouring matter, eg. Bromophenol Blue, to the sample to be assayed as an internal standard, the reproducibility of the migration distance and the accuracy of measurement of the migration distance are considerably enhanced.

The biocomponents assayable by the method of the present invention include: antigens, antibodies, enzymes, enzyme substrates, coenzymes, proteins glycoproteins, glucides, glycolipids, lipoproteins, nucleic acids, hormones, etc. and which are to be found in various body fluids, pathological tissue sections etc. The method of the invention is particularly applicable to the assay of body fluids such as blood, urine, saliva, gastric juice, intestinal juice, pancreatic juice, bile, tissue fluid, edema fluid, ascitic fluid, hydrothorax fluid, hydropericardia fluid, synovial fluid, cerebrospinal fluid, amniotic fluid, genital secretion fluid, mother's milk, nasal mucus, phlegm, tear, sweat, etc., and especially blood serum or plasma.

Also included as target substances are labelled biocomponents comprising as the label an antigen, antibody, enzyme, enzyme substrate, coenzyme, protein, glycoprotein, glucide, glycolipid, lipoprotein, nucleic acid, hormone, avidin or biotin.

As the receptor there may be used a peroxidase conjugate e.g. a peroxidase labelled antibody, protein A, enzyme, enzyme substrate, coenzyme, nucleic acid, hormone-receptor, specifically bound protein (B-2 protein-bound protein, C-reactive protein, etc.), avidin, biotin, or lectin etc. Preferably the receptor is peroxidase (POD)-labelled protein A.

Since the various assay conditions such as the amount of assay sample, the amount and the ratio of enzyme-labelled receptor, the amount of substrate, the reaction time and temperature, etc. depend on the substance to be assayed, the titer of the receptor to be used etc., the most suitable conditions for each assay sample must be selected. For example, when POD-labelled protein A is used in staining the gel after electrophoresis, unbound substances are first eliminated from the gel after electrophoresis, and then a predetermined amount of POD-labelled protein A is added dropwise to the gel surface, followed by allowing the assay sample to stand at approximately room temperature, preferably at 15 to 25°C, for 30 to 45 minutes. Then, the assay sample is washed with an appropriate buffer solution for 30 to 40 minutes, and then hydrogen peroxide and 4-methoxy-1-naphthol are added thereto to initiate the enzyme reaction.

After the precipitate line is thoroughly coloured (usually after 2 to 3 minutes), the assay sample is washed to remove unreacted substances, decolorised and dried. In the washing and decolorisation steps any appropriate reagent may be used, but it is preferable in view of simplicity and rapidity to use running water. After washing and decolorisation, the assay sample is dried slowly since too rapid drying causes unevenness, making the surface of the gel rough and interfering with the assay. Thus, it is preferable to carry out drying at a temperature from room temperature to 60°C.

Preferably the concentration of POD-labelled protein A is adjusted to 0.01 to 0.5% (W/V) by appropriate dilution, the preferred diluting solution being a barbital buffer solution at a pH of about 8.6. The preferred NaCl concentration of the barbital buffer solution is 0.05 to 0.5M. The colouring reagent solution is a solution of about 1% 4-methoxy-1-naphthol in an appropriate solvent, which is further diluted to 0.001 to 0.1% when used. The preferred diluting solution is a Tris-hydrochloric acid buffer solution at a pH of about 7.0 to 8.5.

The method of preparing the gel for the electrophoresis is not particularly critical, and any conventional method can be used. For example, a predetermined amount of agar, agarose or the like is added to distilled water or a suitable buffer such as a barbital buffer solution at a pH of about 8.6 or a Tris-hydrochloric acid buffer solution, and heated at a temperature from 60 to 80°C with gentle stirring to make a solution. The solution is then spread onto a flat plate and left to cool, thereby to coat the plate with the gel.

The gel concentration can be adjusted to the particular characteristics (molecular weight, configuration etc.) of the biocomponent or target substance and the labelled receptor combination and the reaction product (colouring matter). Suitable gel concentrations will usually be 0.5 to 2.0% (W/V), preferably 0.8 to 1.0% (W/V).

An antiseptic may be added to the gel as appropriate.

When assaying a biocomponent of body fluid by gel electrophoresis, it is usual to use a control marker for the migration which is migrated in parallel with the sample. In the conventional method, the migration conditions may well differ between the control marker and the biocomponent due for example to local structural deformations of the gel, unevenness in the gel structure, electrical load conditions, temperature difference, etc., resulting in poor reproducibility. There is also a problem in the accuracy of measurement values.

In accordance with the present invention it has also now been found that measurement values having better accuracy and reproducibility than those of the conventional method can be obtained by using a protein having a different antigen specificity from that of the bio-component, as an internal standard, and incorporating into the carrier gel a receptor capable of being specifically bound to the internal standard protein. In particular, when a mixture of the internal standard substance coloured by a colouring matter (for example, Bromophenol Blue, which will be hereinafter referred to as BPB) with a body fluid is subjected to electrophoresis on a gel-like carrier containing antibodies against the biocomponent and against the internal standard substance, measurement values having a better accuracy and reproducibility can be obtained.

As the internal standard substance usable in the present invention, a protein having no cross reactivity with the biocomponent is employed. Suitable proteins include human serum albumin, bovine serum albumin, egg albumin, chick serum albumin, etc. Particularly preferred are egg albumin and chick serum albumin.

As the receptor for the standard protein to be incorporated into the carrier gel a monoclonal or polyclonal antibody specific to the protein standard is preferably used.

Suitable colouring agents for colouring the internal standard protein include Bromophenol Blue, Bromothymol Blue, etc. To avoid tailing by colouring matter unbound during the migration, it is preferable to adjust the concentrations of these colouring agents to 0.001 to 0.1% (W/V).

In the method of the present invention, the colouring agent, internal standard protein, and body fluid are either mixed directly with one another, or a mixture of the colouring agent and the internal standard protein is first smeared onto a flat plate for reaction and dried, and then the body fluid is added dropwise to the flat plate and mixed with the colouring agent protein conjugate.

The latter procedure is preferred because the concentration of the component in the body fluid does not influence the result, the stability of the internal standard substance is preserved and the technique is simple and rapid.

The present invention is illustrated in, but not to be restricted by, the following Examples and by reference to the accompanying drawings, in which:

Figure 1 shows a standard curve in AFP standard serum assay in Example 1.

Figure 2 shows the result of AFP measurement in amniotic fluids in Example 2.

EXAMPLE 1

Investigation of quantitative determinability of the target substance by rocket electrophoresis:

a) Preparation of AFP standard serum:

AFP standard serum (104l ng/ml, made by DAKO Co. Denmark) was diluted with normal human serum (AFP: 5ng/ml or less) to prepare AFP standard serums (100, 200, 400, 600 and 800 ng/ml).

b) Preparation of anti-AFP-Containing agarose plate:

20 $\mu$l of anti-AFP serum (made by DAKO Co.) were added to 10ml of 1.0% agarose (dissolved in a barbital buffer solution having a pH of 8.6 and an ionic strength of 0.05, made by FMC Marine Colloids Division Co, USA) and mixed. The anti-AFP serum-containing agarose gel was then poured into a gel mould (110 x 125 mm, made by FMC Marine Colloids Division Co.) and coagulated to prepare an anti-AFP-containing agarose plate having a thickness of about 1mm.

c) Preparation of POD-labelled protein A solution:

20 $\mu$l of POD-labelled protein A (made by E Y Laboratories, USA) were added to 10ml of a dilute saline solution (prepared by dissolving 14.0g of NaCl in 1l of barbital buffer solution having a pH of 8.6) to make a 0.2% POD-labelled protein A solution.

d) Preparation of colouring reagent:

Just before application, 200 $\mu$l of 4-methoxy-1-naphthol (made by Aldrich Co, USA), 10ml of 0.05M Tris-hydrochloric acid buffer solution (solution made by mixing aqueous trishydroxymethyl-aminomethane at 12.114 g/l and 0.1M HCl in a ratio of 100:77, pH 7.6) and a drop of 30% $H_2O_2$ were mixed together to make a colouring reagent.

e) Measurement of AFP:

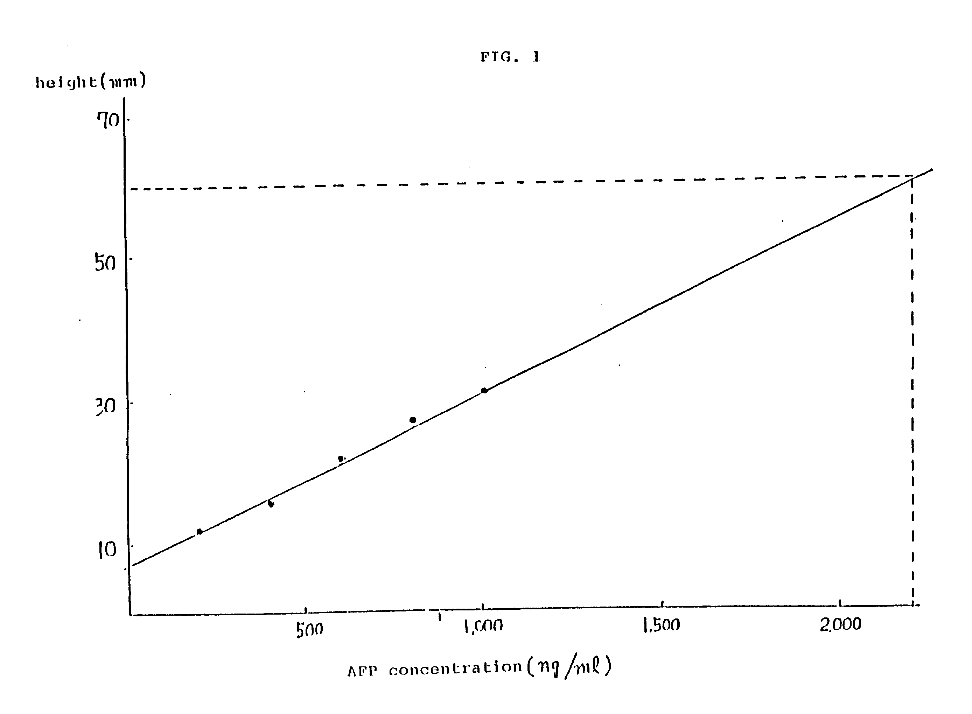

10 $\mu$l each of the AFP standard serums (100, 200, 400, 600, and 800 ng/ml) obtained in said section (a) were poured into holes (4.0mm in diameter) on the anti-AFP-containing agarose plate prepared in said section (b), and subjected to electrophoresis at 3mA/cm for 90 minutes at 10°C. After migration, the plate was treated with an absorbent pad (made by Nihon Shoji Co, Japan) for 2-3 minutes to eliminate unbound substances, and then washed with a phosphate buffer solution (pH 7.2) for 30 minutes. Then, the plate was dried again with an absorbent pad, and 10ml of the POD-labelled protein A solution prepared in said section (c) was poured onto the flat plate. After the reaction had proceeded for 30 minutes, the steps of drying with the absorbent pad, washing with the phosphate buffer solution (pH 7.2) for 30 minutes and drying with the absorbent pad were repeated in the same manner as above. 10ml of the colouring reagent prepared in said section (d) were then added to the plate and reacted for 2 - 3 minutes. Following that the plate was washed with water for 2 to 3 minutes, dried with an absorbent pad and the migration distances measured. By means of this procedure a standard curve is obtained as shown in Figure 1.

EXAMPLE 2

MEASUREMENT OF AFP IN AMNIOTIC FLUID BY CONCANAVALIN-A CROSS-IMMUNOELEC-TROPHORESIS (CAIE):

4

a) Preparation of assay sample:

Amniotic fluids collected from 16 women, 7 to 18 weeks pregnant were centrifuged (3,000 rpm for 20 minutes) and $NaN_3$ was added to the supernatants to a concentration of about 0.001% (W/V). The mixtures were freeze-dried at -20°C to make assay samples.

b) Preparation of Con-A-containing agarose plate:

To 10 ml of 1.0% agarose (dissolved in a barbital buffer solution having a pH of 8.6 and an ionic strength of 0.05) were added 600μg/ml of Con-A (Pharmacia Fine Chemicals Inc. Sweden). 15ml of the Con-A-containing agarose gel were then poured into a gel mould (110 x 125 mm, FMC Marine Colloids Division Co.) and coagulated to make a Con-A-containing agarose plate having a thickness of about 1mm.

c) Measurement of AFP:

10μl aliquots of the samples prepared in section (a) were poured into holes (4mm in diameter) in the Con-A-containing agarose plate prepared in said section (b), and subjected to one-dimensional electrophoresis. Migration was carried out with a barbital buffer solution (pH 8.6) at 3 mA/cm for 90 minutes at 10°C.

As the control, 5 μl of 3% (W/V) human serum albumin [fraction V, made by Sigma Co. USA, containing 0.01% (W/V) BPB] were used and the 6 cm-migration position was marked.

After the one-dimensional electrophoresis, the agarose plate was cut into strips about 1 cm wide parallel to the direction of migration, which were then placed on top of the anti-AFP-containing agarose plate prepared in Example 1, section (b). The assembly was then subjected to further electrophoresis in a direction perpendicular to the first direction at 2.5 mA/cm for 2 hours at 10°C.

After the migration, the plate was treated with an absorbent pad (made by Nichon Shoji Co. Japan) for 2 to 3 minutes, for elimination of unbound substances, and then washed with a phosphate buffer solution (pH 7.2) for 30 minutes. The plate was again dried with an absorbent pad, and reacted with 10 ml of the POD-labelled protein A solution prepared in Example 1, section (c). After the reaction had proceeded for 30 minutes, the steps of drying with an absorbent pad, washing with a phosphate buffer solution (pH 7.2) for 30 minutes and drying with an absorbent pad were repeated in the same manner as above, and then 10 ml of the reagent solution prepared in Example 1, section (d) were added and reacted for 2 to 3 minutes. After washing with water for 2 to 3 minutes and drying with an absorbent pad, the distance from the albumin position obtained in the one-dimensional electrophoresis to a migration peak shown in the two-dimensional electrophoresis was measured.

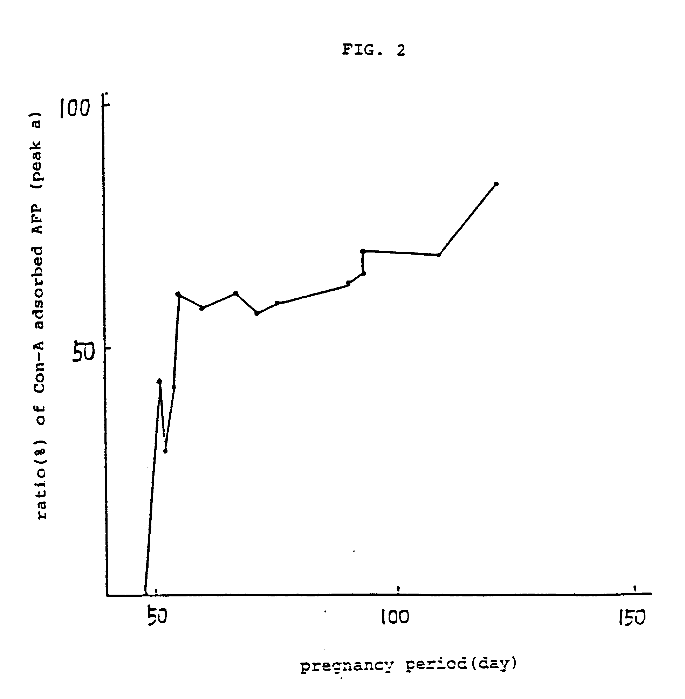

AFP in the amniotic fluid is divided into two fractions by means by Con-A-CAIE. The fraction having an immune precipitate peak at the AFP-migrated position on the lectin-free agarose flat plate by one-dimensional electrophoresis is Con-A unadsorbed AFP (peak b), whereas the peak shown on the cathode side therefrom is Con-A-adsorbed AFP (peak a). Figure 2 shows a graph on which proportions of peaks b are plotted with changes in pregnancy period, defining the total height of peaks a and b as 100%. That is, Figure 2 shows that the Con-A adsorbed AFP in the amniotic fluid tends to increase with the elapse of time.

EXAMPLE 3

MEASUREMENT OF AFP IN THE SERUM OF PATIENTS SUFFERING FROM HEPATIC CARCINOMA BY CON-A-CAIE

a) Preparation of assay sample:

Sera from 47 cases of primary hepatic carcinoma and 8 cases of metastatic hepatic carcinoma were diluted with normal human serum (AFP: 5 ng/ml or less) to prepare assay samples having not more than 15,000 ng/ml of AFP.

b) Measurement of AFP:

Measurement was carried out in the same manner as in Example 2.

As a result, typical migration patterns of sera of patients suffering from hepatic carcinoma by Con-A-CAIE are classified into the type in which a single peak a appears (Type 1), the type in which two fractions, peaks a and b appear (Type 2), and the type in which a single peak b appears (Type 3). As shown in Tables 1 and 2, the AFP in the Serum of primary hepatic carcinoma can be divided into Type 1 (single peak a) and Type 2 (peak a > peak b), whereas that in the metastatic hepatic carcinoma can be divided into Type 2 (peak a < peak b) and Type 3 (single peak b), and thus, this is useful for the differential diagnosis of cancer.

Table 1

| AFP FRACTION APPEARANCE FREQUENCY BY CON-A-CAIE | | | |
|---|---|---|---|
| Fraction Pattern | Fraction as appeared | Primary hepatic carcinoma | Metastatic hepatic carcinoma |
| Type 1 | a | 29 (61.7%) | 0 |
| Type 2 | a, b | 18*(38.3%) | 6 (75%) |
| Type 3 | b | 0 | 2 (25%) |
| | | 47 | 8 |

\* At least two measurements were made for each case, and every case where Type 2 appeared in at least one measurement was classified into Type 2.

Table 2

HEIGHT OF PEAK b AND KIND OF HEPATIC CARCINOMA IN TYPE 2

BY CON-A-CAIE

| | Primary hepatic carcinoma | Metastatic hepatic carcinoma |
|---|---|---|
| Number of investigation | 18 | 6 |
| Height at peak b | | |
| Average % | $14.1 \pm 3.6$[1] | $44.5 \pm 20.2$[2] |
| Range | 8 - 22 | 17 - 68 |

[1]   The higher value was adopted where several measurements were made for each case.

[2]   The lower value was adopted where several measurements were made for each case.

EXAMPLE 4

PROCEDURE FOR MEASURING AFP IN SERUM OF CANCER PATIENT USING CHICK SERUM ALBUMIN (FRACTION V, made by Sigma Co. USA) AS AN INTERNAL STANDARD

a) Preparation of assay sample:

A BPB (made by Sigma Co.) solution was prepared at 0.005% (W/V) with the addition of 0.0025% (W/V) chick serum albumin. 10µl of the BPB solution were smeared onto an agarose plate and dried. A drop (25µl) of serum of a patient suffering from hepatic carcinoma was added to the plate through a Pastem pipette to make an assay sample.

b) Preparation of LCH containing agarose plate:

A plate was prepared in the same manner as in Example 2, section (b) except that lentil lectin (LCH) (Pharmacia Fine Chemicals) was added to 1.0% agarose in place of Con-A to a final concentration of 200 µg/ml.

c) Prepatration of agarose plate for two-dimensional migration:

A plate was prepared according to Example 1, section (b), except that 20 µl of anti-AFP serum (made by DAKO Co.) and 10 µl of anti-chick serum albumin serum (made by Cappel Co. USA) were added to 10 ml of 1.0% agarose.

d) Measurement of AFP:

10µl aliquots of the assay sample prepared in said section (a) were poured into holes in the LCH-containing agarose plate prepared in said section (b) and subjected to one-dimensional electrophoresis under the same conditions as in Example 2, section (c), and the position of BPB marker was marked after the migration.

The agarose plate was cut into strips parallel to the direction of migration and the strips placed on the agarose plate prepared as in said section (c). The assembly was then subjected to electrophoresis under the same conditions as in Example 2, section (c) but in the perpendicular direction and then coloured. Distances to the respective migration peaks from the position of chick serum albumin precipitate peak as the point of origin were measured.

The precipitate peak of chick serum albumin was not crossed with the precipitate peak of serum of a patient suffering from hepatic carcinoma, and formed at the substantially same position as that of BPB marker after the one-dimensional migration.

According to the present invention, biocomponent assays can be carried out with lower background, higher clearness, sensitivity, rapidity, simplicity, reproducibility and accuracy as compared with conventional methods using 4-chloro-1-naphthol as the chromogen. In particular, the measurement sensitivity is about 20-times higher, for example, from 2,000 ng/ml to about 100 ng/ml. Furthermore, higher reproducibility and accuracy can be attained by introduction of an internal standard system and measurement times can be considerably shortened, with a considerable increase in measurement sensitivity and accuracy.

For example, in the case of the Con-A-CAIE of example 2, the two-dimensional migration time, which usually takes 16 hours can be reduced to 2 hours. Also, the colouring time, which takes 30 minutes to one hour according to the conventional method, can be reduced to only 2 to 3 minutes. Furthermore, the antibody concentration during the two-dimensional migration can be enough at 0.15 to 0.3% owing to the increased sensitivity. Moreover the stained gels show no fading and can be permanently preserved. This is a further advantage.

**Claims**

1.  An immunoelectrophoresis assay method, which comprises subjecting a sample containing a biocomponent or a target substance to be assayed to electrophoresis on a gel like carrier and visibly developing the separated band or bands of the biocomponent or target substance to be assayed by treating the gel containing the electrophoresed sample with a peroxidase-labelled receptor conjugate capable of undergoing specific binding to the biocomponent or target substance, washing the treated gel to remove unbound conjugate, and chromogenically developing the bound peroxidase label by reacting the bound label with an enzyme substrate comprising hydrogen peroxide and a chromogen, characterised in that the chromogen used is 4-methoxy-1-naphthol.

2.  A method according to Claim 1, characterised in that the biocomponent to be assayed is an antigen, antibody, enzyme, enzyme substrate, coenzyme, protein, glycoprotein, glucide, glycolipid, lipoprotein, nucleic acid or hormone.

**3.** A method according to Claim 2, characterised in that the biocomponent to be assayed is a $\alpha$-fetoprotein (AFP), human chorionic gonadotropin (HCG), $\Gamma$-glutamyltransferase ($\Gamma$-GTP) or carcinoembryonic antigen (CEA).

**4.** A method according to Claim 1, wherein the target substance to be assayed is a labelled biocomponent containing as the label an antigen, antibody, enzyme, enzyme substrate, coenzyme, protein, glycoprotein, glucide, glycolipid, lipoprotein, nucleic acid, hormone, avidin or biotin.

**5.** A method according to any one of Claims 1-4, characterised in that the peroxidase (POD)-labelled conjugate is a POD-labelled antibody, protein A, enzyme, enzyme substrate, coenzyme, nucleic acid, a hormone-receptor, specifically bound protein, avidin, biotin or lectin.

**6.** A method according to any one of Claims 1-5, characterised in that there is added to the sample prior to electrophoresis, as an internal standard, a coloured protein-dye conjugate, the protein component of which has a different antigenic specificity from that of the biocomponent or target substance to be assayed, and wherein there is incorporated into the gel-like carrier a receptor substance capable of undergoing a specific binding reaction with the standard protein.

**7.** A method according to Claim 6, wherein the protein used as the internal standard is bovine serum albumin, human serum albumin, chick serum albumin or egg albumin and the receptor is the corresponding anti-albumin antibody.

**8.** A method according to Claim 6 or 7, wherein the internal standard protein comprises 0.001 to 0.1% (W/V) colouring agent.

**9.** A method according to any one of Claims 6-8, wherein the internal standard protein is coloured with Bromophenol Blue.

**10.** A diagnostic method which comprises subjecting a clinical sample from a patient having, or suspected of having carcinoma, to an immunoelectrophoresis assay as claimed in any one of Claims 1-7, and using the results of the assay to make or confirm the diagnosis.

**Patentansprüche**

**1.** Immunelektrophoretisches Testverfahren, wobei eine einen zu untersuchenden biologischen Bestandteil oder eine Zielsubstanz enthaltende Probe auf einem gel-ähnlichen Träger einer Elektrophorese unterworfen wird, die aufgetrennte(n) Bande(n) des zu untersuchenden biologischen Bestandteils oder der Zielsubstanz durch Behandlung des die elektrophoretisch aufgetrennte Probe enthaltenden Geles mit einem Peroxidase-markierten Rezeptorkonjugat, das zur spezifischen Bindung zu dem biologischen Bestandteil oder der Zielsubstanz fähig ist, sichtbar entwickelt wird, das behandelte Gel zum Entfernen von ungebundenem Konjugat gewaschen wird, die gebundene Peroxidasemarkierung durch deren Reaktion mit einem Wasserstoffperoxid und ein Chromogen enthaltenden Enzymsubstrat farbgebend entwickelt wird, **dadurch gekennzeichnet**, daß das verwendete Chromogen 4-Methoxy-1-naphthol ist.

**2.** Verfahren nach Anspruch 1, **dadurch gekennzeichnet**, daß der zu untersuchende biologische Bestandteil ein Antigen, Antikörper, Enzym, Enzymsubstrat, Coenzym, Protein, Glykoprotein, Glucid, Glykolipid, Lipoprotein, eine Nucleinsäure oder ein Hormon ist.

**3.** Verfahren nach Anspruch 2, **dadurch gekennzeichnet**, daß der zu untersuchende biologische Bestandteil ein $\alpha$-Fetoprotein (AFP), menschliches Choriongonadotropin (HCG), eine $\Gamma$-Glutamyltransferase ($\Gamma$-GTP) oder ein carcinoembryonales Antigen (CEA) ist.

**4.** Verfahren nach Anspruch 1, wobei die zu untersuchende Zielsubstanz ein markierter biologischer Bestandteil ist, der als Markierung ein Antigen, einen Antikörper, ein Enzym, Enzymsubstrat, Coenzym, Protein, Glykoprotein, Glucid, Glykolipid, Lipoprotein, eine Nucleinsäure, ein Hormon, Avidin oder Biotin enthält.

**5.** Verfahren nach einem der Ansprüche 1 bis 4, **dadurch gekennzeichnet**, daß das Peroxidase (POD)-markierte Konjugat ein POD-markierter Antikörper, Protein A, ein Enzym, Enzymsubstrat, Coenzym, eine Nucleinsäure, ein Hormonrezeptor, ein spezifisch gebundenes Protein, Avidin, Biotin oder ein Lectin ist.

**6.** Verfahren nach einem der Ansprüche 1 bis 5, **dadurch gekennzeichnet**, daß vor der Elektrophorese als interner Standard ein gefärbtes Proteinfarbstoffkonjugat zur Probe gegeben wird, wobei dessen Proteinkomponente eine Antigenspezifität aufweist, die sich von der des zu untersuchenden biologischen Bestandteils oder der Zielsubstanz unterscheidet, und wobei eine zur spezifischen Bindereaktion mit dem Standardprotein fähige Rezeptorsubstanz in dem gelähnlichen Träger eingeschlossen ist.

**7.** Verfahren nach Anspruch 6, wobei das als interner Standard verwendete Protein Rinder-Serumalbumin, Menschen-Serumalbumin, Huhn-Serumalbumin oder Eialbumin und der Rezeptor der entsprechende anti-Albumin-Antikörper ist.

**8.** Verfahren nach Anspruch 6 oder 7, wobei das interne Standardprotein 0,001 bis 0,1 % (Gew./Vol.) eines färbenden Agens enthält.

**9.** Verfahren nach einem der Ansprüche 6 bis 8, wobei das interne Standardprotein mit Bromphenolblau gefärbt ist.

**10.** Diagnostisches Verfahren, **dadurch gekennzeichnet**, daß eine klinische Probe eines krebsverdächtigen Patienten einem immunelektrophoretischen Test nach einem der Ansprüche 1 bis 7 unterworfen wird und daß die Testergebnisse dem Erstellen oder Bestätigen einer Diagnose dienen.

**Revendications**

**1.** Méthode de dosage par immuno-électrophorèse, qui comporte le fait de soumettre un échantillon, contenant un composant biologique ou une substance cible à doser, à une électrophorèse sur un support de type gel, et le fait de développer visiblement la ou les bande(s) séparée(s) du composant biologique ou de la substance cible à doser, en traitant le gel contenant l'échantillon soumis à l'électrophorèse avec un conjugué de récepteur marqué à la peroxydase, capable de se lier spécifiquement au composant biologique ou à la substance cible, en lavant le gel traité pour enlever la fraction non liée de conjugué, et en développant par coloration le marqueur peroxydase lié, par réaction de ce marqueur lié avec un substrat enzymatique comprenant du peroxyde d'hydrogène et un chromogène, la méthode étant **caractérisée** en ce que le chromogène utilisé est le 4-méthoxy-1-naphtol.

**2.** Méthode conforme à la revendication 1, **caractérisée** en ce que le composant biologique à doser est un antigène, un anticorps, une enzyme, un substrat enzymatique, une co-enzyme, une protéine, une glycoprotéine, un glucide, un glycolipide, une lipoprotéine, un acide nucléique ou une hormone.

**3.** Méthode conforme à la revendication 2, **caractérisée** en ce que le composant biologique à doser est une $\alpha$-foetoprotéine (AFP), une gonadotropine chorionique humaine (GCH), une $\gamma$-glutamyltransférase ($\gamma$-GTP) ou un antigène carcino-embryonnaire (ACE).

**4.** Méthode conforme à la revendication 1, dans laquelle la substance cible à doser est un composant biologique marqué, contenant comme marqueur un antigène, un anticorps, une enzyme, un substrat enzymatique, une coenzyme, une protéine, une glycoprotéine, un glucide, un glycolipide, une lipoprotéine, un acide nucléique, une hormone, l'avidine ou la biotine.

**5.** Méthode conforme à l'une quelconque des revendications 1 à 4, **caractérisée** en ce que le conjugué marqué à la peroxydase (POD) est un anticorps, une protéine A, une enzyme, un substrat enzymatique, une co-enzyme, un acide nucléique, un récepteur-hormone, une protéine spécifiquement liée, de l'avidine, de la biotine ou une lectine, marqués à la peroxydase.

**6.** Méthode conforme à l'une quelconque des revendications 1 à 5, **caractérisée** en ce que l'on ajoute à l'échantillon, avant l'électrophorèse, en tant que référence interne, un conjugué coloré de protéine et de colorant, dont le composant protéinique présente une spécificité antigénique différente de celle du composant biologique ou de la substance cible à doser, une substance réceptrice, capable de subir une réaction spécifique de liaison avec la protéine de référence, étant incorporée dans le support de type gel.

7.  Méthode conforme à la revendication 6, dans laquelle la protéine utilisée comme référence interne est de l'albumine sérique bovine, de l'albumine sérique humaine, de l'albumine sérique de poulet ou de l'ovalbumine, et le récepteur est l'anticorps anti-albumine correspondant.

8.  Méthode conforme à la revendication 6 ou 7, dans laquelle la protéine de référence interne contient de 0,001 à 0,1% (p/v) d'un agent colorant.

9.  Méthode conforme à l'une quelconque des revendications 6 à 8, dans laquelle la protéine de référence interne est colorée avec du bleu de bromophénol.

10. Procédé de diagnostic qui comporte le fait de soumettre un échantillon clinique, prélevé sur un patient présentant un cancer ou suspecté d'en présenter un, à un dosage par immuno-électrophorèse, tel que revendiqué dans l'une quelconque des revendications 1 à 7, et l'utilisation des résultats de ce dosage pour établir le diagnostic ou le confirmer.

FIG. 1

height(mm)

AFP concentration (ng/ml)

EP 0 167 340 B1

FIG. 2